(19) Europäisches Patentamt
European Patent Office
Office européen des brevets

(11) Veröffentlichungsnummer : **0 340 645 B1**

(12) # EUROPÄISCHE PATENTSCHRIFT

(45) Veröffentlichungstag der Patentschrift :
**02.09.92 Patentblatt 92/36**

(51) Int. Cl.$^5$ : **C07C 47/445**

(21) Anmeldenummer : **89107674.7**

(22) Anmeldetag : **27.04.89**

(54) **Isomere Formyl-trimethylbicyclo(2,2,2)oct-7-ene.**

(30) Priorität : **05.05.88 DE 3815259**

(43) Veröffentlichungstag der Anmeldung :
**08.11.89 Patentblatt 89/45**

(45) Bekanntmachung des Hinweises auf die Patenterteilung :
**02.09.92 Patentblatt 92/36**

(84) Benannte Vertragsstaaten :
**AT BE CH DE ES FR GB IT LI NL**

(56) Entgegenhaltungen :
**CH-A- 143 252**
**CH-A- 611 517**
**US-A- 3 914 322**

(56) Entgegenhaltungen :
**CHEMICAL ABSTRACTS, Band 97, Nr. 19, 8. November 1982, Seite 741, Spalte 1, Zusammenfassung Nr. 163242x, Columbus, Ohio, US;& JP-A-57 080 391**
**CHEMICAL ABSTRACTS, Band 109, Nr. 15, 10. Oktober 1988, Seite 636, Spalte 1, Zusammenfassung Nr. 128453m, Columbus, Ohio, US;& JP-A-63 122 691**

(73) Patentinhaber : **Henkel Kommanditgesellschaft auf Aktien**
**Postfach 1100 Henkelstrasse 67**
**W-4000 Düsseldorf 1 (DE)**

(72) Erfinder : **Bruns, Klaus, Prof. Dr.**
**Notburgaweg 6**
**W-4150 Krefeld-Traar (DE)**
Erfinder : **Gerke, Thomas, Dr.**
**Im Melchersfeld 6**
**W-4040 Neuss (DE)**
Erfinder : **Virnig, Michael, Dr.**
**1105 Lanewood Way 15**
**Santa Rosa, CA 95404 (US)**

**EP 0 340 645 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft isomere Formyl-trimethylbicyclo[2.2.2]oct-7-ene, deren Herstellung sowie deren Verwendung als Riechstoffe.

Aus der Literatur sind bereits Bicyclo[2.2.2]octene als Riechstoffe bekannt. So werden beispielsweise in Chem. Abstr. 76, 4021z (1972) Bicyclooctene offenbart, die mit einer Isopropylgruppe substituiert sind. In CH 611 517 werden substituierte Tetramethylbicyclo[2.2.2]octene beschrieben.

Es wurde nun gefunden, daß isomere Formyl-trimethylbicyclo[2.2.2]oct-7-ene überraschende und wertvolle Riechstoffeigenschaften besitzen.

Erfindungsgegenstand sind dementsprechend isomere Formyl-trimethylbicyclo[2.2.2]oct-7-ene der allgemeinen Formeln

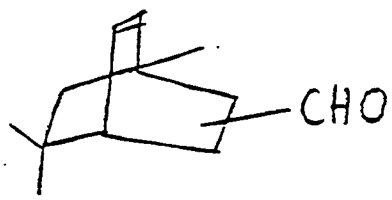

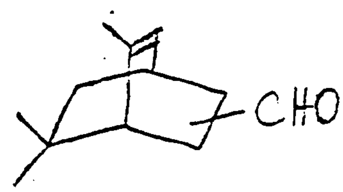

Ferner ist die Verwendung dieser isomeren Formyl-trimethylbicyclo[2.2.2]oct-7-ene als Riechstoffe Gegenstand der Erfindung.

Der Geruch der erfindungsgemäßen Isomerengemische läßt sich als grün und krautig mit einer Gras- und Heu-Note beschreiben. Des weiteren zeichnen sich die erfindungsgemäßen Gemische durch eine außerordentlich gute Beständigkeit des Geruchsbildes aus.

Isomere Formyl-trimethylbicyclo[2.2.2]oct-7-ene eignen sich zur Herstellung neuer, interessanter Riechstoffkompositionen, in denen frisch-krautige Duftnoten gefordert werden. Bezogen auf die gesamte Komposition liegt der Gehalt der erfindungsgemäßen Riechstoffgemische zwischen 1 und 50 Gew.-%, vorzugsweise zwischen 1 und 25 Gew.-%. Diese Kompositionen können zur Parfümierung von technischen Artikeln, wie Reinigungs- und Desinfektionsmitteln, von Textilbehandlungsmitteln, von Kosmetika aller Art, wie Duftwässern, Cremes, Lotionen, Aerosolen, Toilettenseifen, Schminken und Lippenstiften sowie in der Extraitparfümerie eingesetzt werden. In den parfümierten Produkten liegt der Gehalt der Riechstoffkompositionen zwischen 2 und 20 Gew.-%, vorzugsweise zwischen 5 und 10 Gew.-%.

Die isomeren Formyl-trimethylbicyclo[2.2.2]oct-7-ene werden in an sich bekannter Weise mittels Diels-Alder-Reaktion hergestellt (s. Wollweber in Houben-Weyl, Bd. V/1c, S. 977 - 1139 (1970)), in dem 1,5,5-Trimethyl-cyclohexa-1,3-dien, 3,5,5-Trimethyl-cyclohexa-1,3-dien, 5,5-Dimethyl-3-methylen-cyclohex-1-en oder Gemische, enthaltend solche Diene, mit Acrolein bei 20 bis 220 °C, gegebenenfalls in Gegenwart von Katalysatoren umgesetzt werden. Das molare Verhältnis Dien : Acrolein liegt zwischen 0,5 und 2, vorzugsweise zwischen 0,6 und 1,5. Als Katalysatoren eignen sich beispielsweise $BF_3$, $AlCl_3$ und/oder $ZnCl_2$. Die Reaktion wird vorzugsweise in organischen Lösungsmitteln, wie Methylenchlorid, Diethylether und/oder Cyclohexan, durchgeführt. Nach beendeter Reaktion wird mit Wasser gewaschen, mit beispielsweise wäßriger Natriumhydrogencarbonatlösung, wäßriger Natriumcarbonatlösung und/oder Kalilauge neutralisiert und anschließend destilliert.

Die als Edukte zur Herstellung der erfindungsgemäßen Bicyclooctene einzusetzenden Diene können nach in der organischen Chemie bekannten und üblichen Methoden hergestellt werden, indem beispielsweise 3,5,5-Trimethylcyclohexen-2-on-1 (Isophoron) mit beispielsweise Lithiumaluminiumhydrid zu dem entsprechenden Alkohol 3,5,5-Trimethylcyclohexenol-1 reduziert wird. Dieser Alkohol wird anschließend mit beispielsweise Kaliumhydrogensulfat zu Dienen umgesetzt, die erfindungsgemäß als Edukte zur Herstellung isomerer Formyl-trimethylbicyclo[2.2.2]oct-7-ene eingesetzt werden, können.

### Beispiele

#### 1. Reduktion von Isophoron

Unter Rühren wurden 200 g Isophoron (1,45 Mol) in 500 ml absolutem Diethylether zu 13,77 g Lithiumaluminiumhydrid (0,3625 Mol, in 200 ml absolutem Diethylether suspendiert) so zugetropft, daß ständiges Sieden am Rückfluß erfolgte. Danach wurde noch eine Stunde unter Rückfluß gekocht. Zur Aufarbeitung wurde nach Abkühlen unter Rühren vorsichtig zunächst mit Eiswasser, dann mit 10 %iger Schwefelsäure versetzt, im

Scheidetrichter getrennt und noch dreimal ausgeethert.

Nach Waschen der organischen Phasen mit gesättigter Kochsalzlösung wurde eingeengt und im Wasserstrahlvakuum (16 Torr) destilliert (60 - 62 °C).

Ausbeute: 170 g (84 % der Theorie)

2. Herstellung des Diengemisches

170 g 3,5,5-Trimethylcyclohexenol, erhalten durch Reduktion von Isophoron, wurden mit 10 g Kaliumhydrogensulfat unter Stickstoff zum Sieden erhitzt, wobei das Diengemisch abdestillierte. Es wurde nach Trocknung mit Natriumsulfat direkt in die Diels-Alder-Reaktion eingesetzt.

3. Herstellung von 5(6)-Formyl-1,3,3(3,3,7)-trimethylbicyclo[2.2.2]oct-7-en durch Diels-Alder-Reaktion

122 g (1 Mol) des nach 2. erhaltenen Diengemisches, enthaltend 1,5,5-Trimethyl-cyclohexa-1,3-dien, 3,5,5-Trimethyl-cyclohexa-1,3-dien und 5,5-Dimethyl-3-methylen-cyclohex-1-en im Gewichtsverhältnis 55 : 25 : 20, 84 g (1,5 Mol) Acrolein und 6,11 g Zinkchlorid wurden in 200 ml Methylenchlorid gelöst und 4 Stunden unter Rückfluß gekocht.

Zur Aufarbeitung wurde mit 100 ml Wasser gewaschen, mit 100 ml einer wäßrigen gesättigten Natriumhydrogencarbonatlösung neutralisiert und nach Entfernen des Lösungsmittels mittels Destillation gereinigt.

Es wurden 115 g = 65 % der Theorie isomeres 5(6)-Formyl-1,3,3(3,3,7)-trimethybicyclo[2.2.2]oct-7-en mit einem Siedepunkt von 58 - 60 °C/0,08 mbar erhalten.

Kompositionsbeispiele:

Phantasie-Lavendel

| | |
|---|---|
| 5(6)-Formyl-1,3,3(3,3,7)-trimethylbicyclo[2.2.2]oct-7-en | 400 Gew.-Teile |
| Lavandinöl Grosso | 200 Gew.-Teile |
| α-Terpineol | 100 Gew.-Teile |
| Linalylacetat | 100 Gew.-Teile |
| Linalool | 50 Gew.-Teile |
| Moschus Keton | 40 Gew.-Teile |
| Lavendelöl franz. | 30 Gew.-Teile |
| Cumarin | 20 Gew.-Teile |
| Sandel (Haarmann & Reimer) | 20 Gew.-Teile |
| Patchouliöl | 20 Gew.-Teile |
| Campher | 15 Gew.-Teile |
| Ambroxan (Henkel) 10 Gew.-%ig in Isopropylmyristat | 5 Gew.-Teile |
| | 1000 Gew.-Teile |

Formulierung für ein Schaumbad/Shampoo

| | |
|---|---|
| 5(6)-Formyl-1,3,3(3,3,7)-trimethylbicyclo[2.2.2]oct-7-en | 200 Gew.-Teile |
| Fichtennadelöl sibir. | 100 Gew.-Teile |
| Isobornylacetat | 80 Gew.-Teile |
| α-Amylzimtaldehyd | 80 Gew.-Teile |
| Phenylethylalkohol | 70 Gew.-Teile |
| Lavandinöl grosso | 70 Gew.-Teile |
| p-tert.-Butylcyclohexylacetat | 60 Gew.-Teile |
| Nopylacetat | 50 Gew.-Teile |
| Orangenöl süß | 50 Gew.-Teile |
| Cyclovertal (Henkel) 10 Gew.-% in Dipropylenglycol | 40 Gew.-Teile |
| Cedrylacetat | 40 Gew.-Teile |
| Galaxolide (IFF) | 40 Gew.-Teile |
| Geraniol | 30 Gew.-Teile |
| Geranylnitril | 30 Gew.-Teile |
| Styarallylacetat | 20 Gew.-Teile |
| Galbanum Resin synth. (RBD) | 20 Gew.-Teile |
| Phenylacetaldehyddimethylacetal | 10 Gew.-Teile |
| Cyclohexylsalicylat (Henkel) | 10 Gew.-Teile |
| | 1000 Gew.-Teile |

## Patentansprüche

1. Isomere Formyl-trimethylbicyclo[2.2.2]oct-7-ene der allgemeinen Formeln

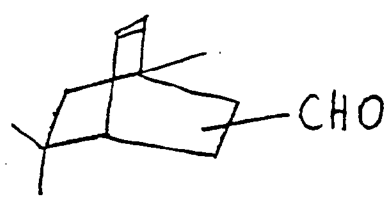

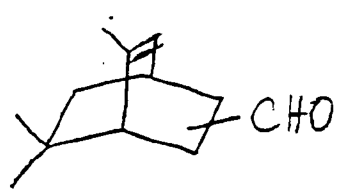

2. Verfahren zur Herstellung isomerer Formyl-trimethylbicyclo[2.2.2]oct-7-ene nach Anspruch 1, dadurch gekennzeichnet, daß man 1,5,5-Trimethyl-cyclohexa-1,3-dien, 3,5,5-Trimethyl-cyclohexa-1,3-dien, 5,5-Dimethyl-3-methylen-cyclohex-1-en oder Gemische, enthaltend diese Diene, mit Acrolein bei Temperaturen zwischen 20 und 220 °C, gegebenenfalls in Gegenwart von Katalysatoren umsetzt, wobei das molare Verhältnis Dien : Acrolein zwischen 0,5 und 2 liegt.

3. Verwendung isomerer Formyl-trimethylbicyclo[2.2.2]oct-7-ene der allgemeinen Formeln nach Anspruch

1 als Riechstoffe.

## Claims

1. Isomeric formyl trimethylbicyclo[2.2.2]oct-7-enes corresponding to the following general formulae

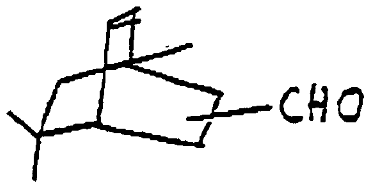

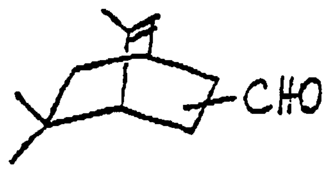

2. A process for the production of the isomeric formyl trimethylbicyclo[2.2.2]oct-7-enes claimed in claim 1, characterized in that 1,5,5-trimethylcyclohexa-1,3-diene, 3,5,5-trimethylcyclohexa-1,3-diene, 5,5-dimethyl-3-methylene cyclohex-1-ene or mixtures containing these dienes are reacted with acrolein at temperatures of 20 to 220°C, optionally in the presence of catalysts, the molar ratio of diene to acrolein being between 0.5 and 2:1.

3. The use of the isomeric formyl trimethylbicyclo[2.2,2]oct-7-enes corresponding to the general formulae in claim 1 as fragrances.

## Revendications

1°) Formyl-triméthylbicyclo(2,2,2)oct-7-ènes isomères, de formules générales :

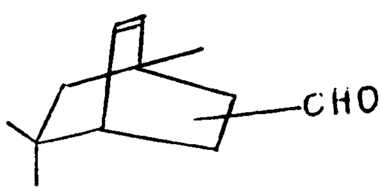

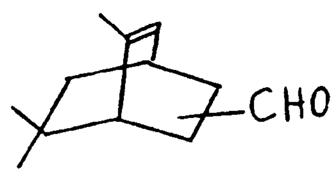

2°) Procédé d'obtention des formyl-triméthylbicyclo(2,2,2)oct-7-ènes isomères selon la revendication 1, caractérisé en ce que l'on met en réaction le 1,5,5-triméthyl-cyclohexa-1,3-diène, le 3,5,5-triméthyl-cyclohexa-1,3-diène et le 5,5-diméthyl-3-méthylène-cyclohex-1-ène ou leurs mélanges contenant ces diènes, avec de l'acroléine à des températures entre 20 et 220°C, le cas échéant en présence de catalyseurs, le rapport molaire diène/acroléine se situant entre 0,5 et 2.

3°) Utilisation des formyl-triméthylbicyclo-(2,2,2)oct-7-ènes de formules générales selon la revendication 1, comme parfums.